# EUROPÄISCHE PATENTSCHRIFT

(11) **EP 1 575 392 B1**
(45) Veröffentlichungstag und Bekanntmachung des Hinweises auf die Patenterteilung: **17.10.2007**
(21) Anmeldenummer: 03769142.5
(22) Anmeldetag: 12.11.2003
(51) Int. Cl.: A44C 7/00, A61B 5/107

(54) **OHRSCHMUCK-SYSTEM MIT ZUGEHÖRIGER LEHRE ZUR OPTIMALEN ANPASSUNG DES OHRSCHMUCKS AN DAS OHRLÄPPCHEN**
EARRING SYSTEM WITH ASSOCIATED TEMPLATE FOR OPTIMAL ADAPTATION OF THE EARRING TO THE EAR LOBE
SYSTEME DE BOUCLE D'OREILLE AVEC GABARIT ASSOCIE POUR UNE ADAPTATION OPTIMALE DE LA BOUCLE D'OREILLE AU LOBE DE L'OREILLE

(30) Priorität: 19.11.2002 CH 193502
(43) Veröffentlichungstag der Anmeldung: 21.09.2005
(73) Patentinhaber: Durrer, Erich M., 6048 Horw (CH)
(72) Erfinder: Durrer, Erich M., 6048 Horw (CH)
(74) Vertreter: Felber, Josef
(86) Internationale Anmeldenummer: PCT/CH2003/000741
(87) Internationale Veröffentlichungsnummer: WO 2004/045325

(56) Entgegenhaltungen:
- DE-B- 1 279 282
- GB-A- 609 457
- US-A1- 2001 011 421

## Beschreibung

Die Erfindung betrifft ein Ohrschmuck-System mit einer ganz speziellen Art von Ohrschmuck, die sich durch das besondere Befestigungssystem auszeichnet. Desweiteren schliesst die Erfindung Lehren ein, damit der Ohrschmuck nach diesem System individuell an ein Ohrläppchen anpassbar ist, was eine wichtige Vor aussetzung für den Tragkomfort des Ohrschmucks ist. Das System ist geeignet für Modeschmuck wie auch für echten Schmuck.

Bei der herkömmlichen Befestigung von Ohrschmuck werden grundsätzlich zwei Arten der Befestigung unterschieden. Nach der ersten Art kann der Schmuck an einem in das Ohrläppchen gestochenen Loch befestigt werden. Auch andere Körperteile werden zuweilen in ähnlicher Weise perforiert, zum Beispiel die Ohrränder oder die Nasenflügel, um daran einen Schmuckring anzuhängen. Das Schmuckstück oder eben der Ohrschmuck weist dann eine Nadel auf, die durch das Loch geschoben wird und an ihrer Spitze mit einem Verschluss, etwa in Form einer Sicherungsklammer, vor dem Herausfallen gesichert wird. Dieser Verschlussmechanismus ist oft unansehnlich. Er muss ja von einer bestimmten Grösse sein, damit er noch ohne Mühe bedient werden kann, und deshalb bleibt er in vielen Fällen von hinten sichtbar, was nicht richtig überzeugt.

Die zweite Befestigungsart besteht darin, den Schmuck an das Ohr oder den Nasenflügel zu klemmen. Der als Ohrclips bezeichnete Ohrschmuck besteht zum Beispiel aus einer Klemmvorrichtung, deren beide Klemmteile mit einer Feder gegeneinander gedrückt werden. Zum Anlegen des Ohrschmucks wird der Ohrclips geöffnet, indem die beiden Klemmteile gegen die Federkraft auseinandergespreizt werden, wonach der Ohrschmuck über das Ohrläppchen gestülpt wird und dann die beiden Klemmteile das zwischen ihnen liegende Ohrläppchen einklemmen. Der Ohrschmuck hält dann aufgrund der Haftreibung am Ohrläppchen. Je grösser und schwerer der Ohrschmuck ist, um so stärker muss der Anpressdruck der Klemmteile und somit die Federkraft sein, um den Ohrschmuck sicher am Ohr festzuklemmen. Bei zu kleiner Federkraft wäre die Haftreibung zu gering und bei abrupten Kopfbewegungen würde der Ohrschmuck vom Ohr abrutschen. Diese Befestigungsmethode ist zwar praktisch zum An- und Ablegen des Ohrschmucks, hat aber den Nachteil, dass sie praktisch immer einen gewissen Schmerz verursacht, vorallem wenn der Ohrschmuck permanent getragen wird. Die Klemmvorrichtungen sind meist Standardausführungen, die einen solchen Anpressdruck erzeugen, dass jede gängige Schmuckart einigermassen sicher gehalten wird. Nach ein paar Minuten des Tragens klingt der durch die Klemmung verursachte Schmerz ab, sodass er nicht mehr über Gebühr stört. Bei längerem Tragen aber macht er sich unweigerlich wieder störend bernerkbar. So gibt es bis heute keinen durch Federkraft anklemmbaren Ohrschmuck für Ohrläppchen, welcher das langanhaltende schmerzfreie und sichere Tragen von Ohrschmuck erlaubt. Je grösser und schwerer der angehängte Schmuck ist, umso gravierender tritt dieses Problem zutage. Weil das Tragen von Ohrclips bisher ausnahmslos mit einer mehr oder weniger starken Schmerzempfindung verbunden ist, verzichten Viele auf das Tragen eines solchen Ohrschmucks, obgleich sie ihn aus ästhetischen Gründen attraktiv fänden und gerne anlegen würden.
Dokument GB-A-609 457 offenbart einen Ohrschmuck mit einer U- förmigen Ausnehmung.

Es ist deshalb die Aufgabe der vorliegenden Erfindung, ein Ohrschmuck-System zu schaffen, welches gewährleistet, dass der Ohrschmuck einfach in der Handhabung zum Anlegen und Abziehen ist, welches weiter auch beim langanhaltenden Tragen von grösserem und schwererem Schmuck keine Schmerzempfindungen auslöst und das darüber hinaus ein Befestigungssystem einsetzt, das einfacher und kostengünstiger herstellbar ist als herkömmliche federbelastete Klemmvorrichtungen und welches bei angelegtem Ohrschmuck von keiner Seite einsehbar ist. In einer Variante soll das System auch eine zusätzliche Sicherung vor dem versehentlichen Abstreifen oder dem bewussten Abstreifen des Ohrschmuckes durch Diebe bieten. Der Ohrschmuck nach diesem System soll individuell an das jeweilige Ohrläppchen der Trägerin angepasst auswählbar sein, wobei standardisierte Grössen für die verschiedenen Ohrläppchengrössen wählbar sind und sodann auf Anhieb zum jeweiligen Ohrläppchen passen.

Diese Aufgabe wird gelöst von einem Ohrschmuck-System mit zugehöriger Lehre aus Einzellehren zur optimalen Anpassung des Ohrschmucks an das Ohrläppchen, welches sich dadurch auszeichnet, dass der Ohrschmuck eine U-förmige Ausnehmung aufweist, welche dazu bestimmt ist, mit ihrer lichten Weite oder mit der lichten Weite einer in sie einzulegenden Einlage von unten über das zu bestückende, für die Zwecke des Anlegens gedehnte Ohrläppchen geschoben zu werden und dabei genau die Dicke dieses gedehnten Ohrläppchens aufzunehmen, wobei die passende lichte Weite mittels einer zum System gehörigen Lehre aus einer Mehrzahl von Einzellehren mit je Mäulern von abgestuften lichten Weiten und Zungen der Dicke der jeweiligen lichten Weiten bestimmbar ist, sodass anhand einer Messung eines zu bestückenden Ohrläppchens im gedehnten Zustand mittels dieser Einzellehren die passende lichte Weite der U-förmigen Ausnehmung des zugehörigen Ohrschmucks oder der einzulegenden Einlage aus einer Ohrschmuck-Reihe oder Einlagen-Reihe mit U-förmigen Ausnehmungen unterschiedlicher lichter Weiten bestimmbar ist.

Der grundsätzliche Kern des Systems besteht darin, dass erstens die Ohrläppchen-Dicke im durch Ziehen gedehnten Zustand des Ohrläppchens individuell gemessen wird und zweitens der Ohrschmuck mit einer genau zu einer in diesem gedehnten Zustand bestimmten Ohrläppchendicke passenden Ausnehmung rein aufgrund von Adhäsionskräften am Ohrläppchen gehalten wird, nachdem das Ohrläppchen nach Überstülpen des Ohrschmucks und Absetzen der Ziehkraft wieder zurückquillt. Die individuelle Messung erfolgt vorteilhaft mit einer standardisierten Skala, sodass ein Ohrläppchen mit einer bekannten Masseinheiten-Dicke auf Anhieb mit einem Ohrschmuck der zugehörigen Weite der U-förmigen Ausnehmung bzw. deren Einlage an dieses Ohrläppchen im gedehnten Zustand passt und nach Absetzen der Dehnung an ihm sicher gehalten ist. Dieses System zum Festhalten des Schmuckes an einem Ohrläppchen ist von aussen nicht sichtbar und somit ist das Haltesystem vollständig in das Innere des Schmuckes integriert. Es versteht sich, dass das System auch bloss als Halterung zum Anhängen des eigentlichen Ohrschmuckes ausgefünrt sein kann.

Eine beispielsweise Ausführung dieses Ohrschmucks-Systems ist in den Zeichnungen dargestellt und das Ohrschmucksystem mit einigen Ohrschmuckstücken als Beispiel sowie mit der zum System gehörigen Lehre aus abgestuften Einzellehren wird im Folgenden beschrieben und die Funktion des Systems wird in allen Einzelheiten und Aspekten erklärt.

### Es zeigt:

- Figur 1:: Das Ohrschmuck-System mit einem einzelnen Ohrschmuckstück und einer zum System gehörigen Lehre aus Einzellehren;
- Figur 2:: Eine Reihe von Ohrschmuckstücken des gleichen Typs mit U-förmigen Ausnehmungen unterschiedlicher lichter Weiten und daneben jeweils die zugehörige Einzellehre;
- Figur 3:: Ein Ohrschmuckstück nach diesem Ohrschmuck-System in einer vergrösserten Darstellung mit einer seiner U-förmigen Ausnehmung;
- Figur 4:: Die Ausmessung der Dicke eines mit einem Ohrschmuck zu bestückenden Ohrläppchens im durch Ziehen gedehnten Zustand;
- Figur 5:: Die Bestimmung des zur ausgemessenen Dicke des Ohrläppchens passenden Ohrschmuck-Stücks;
- Figur 6:: Ein Ohrschmuckstück mit zugehöriger Einlage zur Anpassung an die ausgemessene Dicke eines Ohrläppchens im gedehnten Zustand;
- Figur 7:: Ein auf ein Ohrläppchen aufgeschobenes Ohrschmuckstück, das mit Steinen besetzt ist;
- Figur 8:: Ein auf ein Ohrläppchen aufgeschobenes Ohrschmuckstück in Form einer Kreole;
- Figur 9:: Ein Ohrschmuckstück nach diesem Ohrschmuck-System in einer vergrösserten Darstellung seiner U-förmigen Ausnehmung und einer zusätzlichen Einrichtung zur Sicherung gegen das Abstreifen des Ohrschmucks.

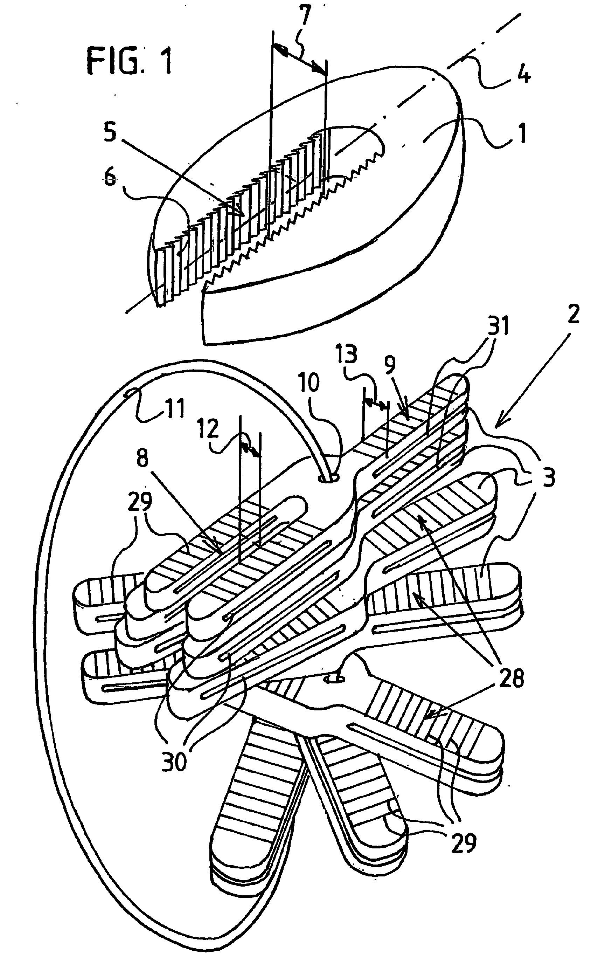

Die Figur 1 zeigt das Ohrschmuck-System mit den wesentlichen Bestandteilen, nämlich mit einem einzelnen Ohrschmuckstück 1 und darunter dargestellt einer zum System gehörigen Lehre 2 aus einer Anzahl Einzellehren 3. Das Ohrschmuckstück 1 bildet hier einen schlichten Schmuck aus einem geeigneten Material, sei es aus Edelmetall, Edelstahl, Aluminium, Kunststoff oder gar aus Stein, Holz oder Glas. Der Ohrschmuck bildet im gezeigten Beispiel eine ellipsenförmige Scheibe von etwa 2 bis 3mm Stärke, etwa 25mm Länge und 15mm Breite. Aus dieser ellipsenförmigen Scheibe ist von einer spitzen Seite der Ellipse her längs ihrer Längsachse 4 eine U-förmige Ausnehmung 5 ausgenommen. Die Ausnehmung 5 weist hier eine fein gezahnte Oberfläche auf, sodass eine ganze Reihe von länglichen Widerhaken gebildet werden. Die Widerhaken können auch als Aufnahmeführung für eine entsprechend ausgebildete Einlage 25 dienen, welche von den Widerhaken zurückgehalten wird, oder bei hohl gearbeiteten Ohrschmuckstücken 1 können die Widerhaken teilweise geöffnet sein, damit das zurückquillende Ohrläppchen noch mehr Platz findet und sich teilweise im Innern des Schmuckstückes zusätzlich verankern kann. Als Alternative kann die Ausnehmung auch glatte oder durch eine Ätzung raue Innenwände 6 aufweisen, ähnlich wie die Reibflächen einer Zündholzschachtel. Dieses Ohrschmuckstück 1 kann aus Vollmaterial gefertigt sein oder auch ein Hohlkörper sein. Das Letztere kann so zustande kommen, dass das Ohrschmuckstück 1 herstellungsbedingt zunächst auf seiner innern, der Ausnehmung 5 zugewandten Seite offen bleibt und diese offenen Seiten hernach mittels eines dünnen Materialstreifens verschlossen werden, entweder durch Einlöten, Einschweissen, Einklicken, Einschieben oder Einkleben entsprechender bandförmiger Streifen. Als Besonderheit muss nun die lichte Weite 7 der Ausnehmung 5 gemäss diesem speziellen Ohrschmuck-System exakt an die Dicke eines Ohrläppchens angepasst sein, und zwar an die Dicke eines Ohrläppchens in dessen gedehntem Zustand, wenn es mit einer Hand nach unten in die Länge gezogen wird. Der Ohrschmuck wird deshalb in einer ganzen Reihe hergestellt, wobei sich die einzelnen Ohrschmuckstücke 1 einer Schmuckreihe durch das Mass ihrer lichten Weiten 7 unterscheiden. Die Ohrschmuckstücke 1 werden also mit einer ganzen Reihe abgestufter Weiten 7 hergestellt, genau wie etwa auch Fingerringe eines spezifischen Schmucktyps in unterschiedlichen Grössen bzw. Durchmessern gefertigt werden. Die U-förmige Ausnehmung 5 am Ohrschmuck ist etwa 10mm bis 15mm lang und ihre lichte Weite 7 beträgt zwischen 2mm und 8mm. Die meisten Ohrschmuckträgerinnen werden eine Weite 7 von zwischen 4mm und 6mm benötigen. Die geringsten Weiten 7 werden von jungen Mädchen im Kindesalter benötigt und die grössten Weiten von korpulenten Personen. Eine Reihe eines Ohrschmucktyps kann sodann in Abstufungen der lichten Weiten 7 hergestellt werden, also mit Weiten von 2mm, 2.5mm, 3mm, 3.5mm, ....., 7.5mm, 8mm. Selbstverständlich sind noch feinere Abstufungen wählbar. Unterhalb des Ohrschmucks 1 ist in Figur 1 eine zugehörige Lehre 2 dargestellt. Diese besteht aus einer Anzahl loser Einzellehren 3, welche alle je ein Maul 8 und eine Zunge 9 aufweisen und die hier aufeinandergestapelt dargestellt sind. Damit die Einzellehren 3 nicht verloren gehen, können sie ein Loch 10 aufweisen und dann von einem Drahtring 11 gehalten sein, der durch die Löcher 10 sämtlicher Einzellehren 3 verläuft, wobei diese in der Reihenfolge ihrer abgestuften Weiten 12 bzw. Grössen aneinandergereiht sind. Die lichte Weite 12 des Mauls 8 entspricht bei jeder Einzellehre 3 exakt der Breite 13 der Zunge 9. Oben und unten sind die Einzellehren mit einer Skalierung 28 ausgestattet, das heisst mit feinen Strichen 29, die zum Beispiel 1 mm voneinander beabstandet sind. Ausserdem erstreckt sich durch jede Einzellehre 3 ein Schlitz, und zwar einerseits durch ihr Maul 8 ein Schlitz 30 und andrerseits durch ihre Zunge 9 ein Schlitz 31, der sich bis zur Zungenspitze erstreckt und dort also offen ist. Die Funktion der Skalierung 28 und der Schlitze 30,31 wird im Zusammenhang mit der Beschreibung der Funktion des Systems noch erläutert. Weniger präzise Lehren können aus einem Karton gestanzt sein oder in einem Kunststoffteil gespritzt werden, wobei die Einzellehren nebeneinander liegen. Solchermassen hergestellte Lehren eignen sich besonders gut für den Vertrieb im Versandhandel und für den Gelegenhe'itsgebrauch des Schmucks in privaten Haushalten, da sie sehr kostengünstig sind. Bereits heute werden ähnlich flache Lehren zum Bestimmen der Ringgrösse von Fingerringen eingesetzt.

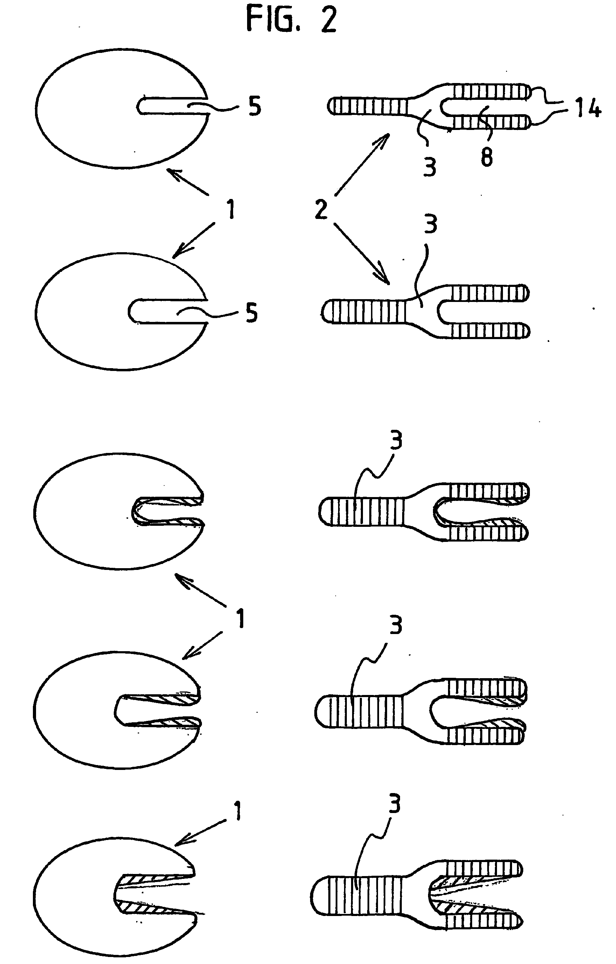

Die Figur 2 zeigt zum besseren Verständnis des Ohrschmuck-Systems die ganzen Bestandteile eines kompletten möglichen Sets eines Ohrschmucktyps. Es besteht also aus einer ganzen Reihe von Ohrschmuckstücken 1 des gleichen Typs mit U-förmigen Ausnehmungen 5 unterschiedlicher lichter Weiten und den zu diesen unterschiedlichen Weiten zugehörigen Einzellehren 3. Die Einzellehren 3 werden vorteilhaft in Kunststoff oder Holz hergestellt, sodass sie leicht sind und nur eine geringe Wärmekapazität und Wärmeleitfähigkeit besitzen, sodass beim Anlegen an ein Ohrläppchen wie das noch beschrieben wird praktisch kein Wärmeübergang stattfindet. Das Anlegen an ein auszumessendes Ohrläppchen ist so für die Probandin viel angenehmer als es etwa mit einer Lehre aus Stahl wäre, die sofort viel Wärme aus dem Ohrläppchen abziehen würde. Die Enden 14 der Lehren 3 auf der offenen Seite ihrer Mäuler 8 sind abgerundet, um beim Aufstecken ein Ohrläppchen nicht zu stechen oder zu kratzen. Wie in den drei untersten Einzellehren 3 angedeutet, kann deren Maul 8 auch eine bestimmte Innenform auf weisen, um der Tatsache Rechnung zu tragen, dass auch die Ohrläppchen über ihre Länge nicht alle eine gleichmässige Dicke aufweisen, sondern etwa nach unten dicker oder dünner werden, etc. Trot-zdem können die Innenseiten dieser Ausnehmungen mit hier nicht eingezeichneten feinen Widerhaken versehen sein, wie das in Figur 1 gezeigt ist, oder diese Innenseiten können sonstwie eine raue Oberfläche aufweisen. Für jede Ohrläppchenform kann ein Set von Einzellehren 3 zur Verfügung gestellt werden. Gleichermassen können auch die Innenformen der Ausnehmungen 5 an den Ohrschmuckstücken 1 verschiedenartig geformt sein, wie an den untersten drei Ohrschmuckstücken 1 mit den schraffierten Bereichen angedeutet.

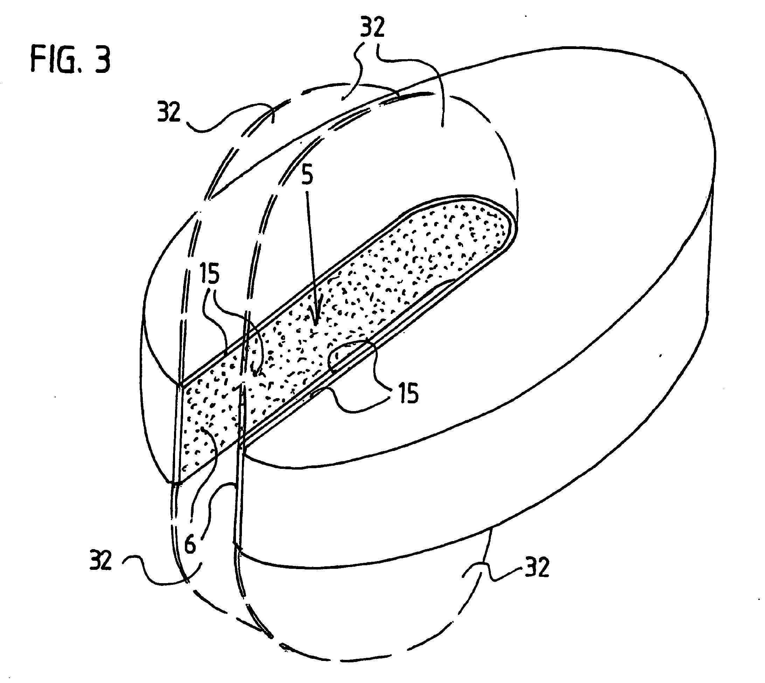

In Figur 3 ist ein Ohrschmuckstück nach diesem Ohrschmuck-System in einer vergrösserten Darstellung mit seiner U-förmigen Ausnehmung 5 gezeigt. Die Innenwände 6 der U-förmigen Ausnehmung 5 können je nach Materialbeschaffenheit des Schmuckstücks frei bleiben oder werden mit einer Gummifolie oder einem gummielastischen Folienmaterial 15, zum Beispiel mit einem Folienmaterial aus einem Neopren-Material bestückt, wozu dann wiederum eine raue Oberfläche oder eine gar mit Widerhaken versehen Oberfläche dienlich ist. Das einzusetzende Material besteht aus einem Trägergewebe aus 100% Polyamid und ist mit einem dehnbaren Kunstkäutschuk beschichtet. Die Innenwände 6 der U-förmigen Ausnehmung 5 werden dann mit einer solchen Folie 15 aus diesem oder ähnlichem Material durch Aufkaschieren bestückt. Solches Folienmaterial weist gegenüber der Haut einen hohen Reibungswiderstand auf und gewährleistet einen sicheren Halt des auf ein Ohrläppchen aufgesteckten Schmuckes. Wie in der Figur 3 strichliniert angedeutet, kann die Folie 15 selbst auch als Teil des Schmuckes gestaltet sein, indem sie die Ausnehmung 5 auf einer oder beiden Seiten überragt und eine zum Beispiel fächerförmige Verzierung 32 bildet. In einer Variante kann die Ausnehmung auch mit einer Gummifolie mit einer speziellen Oberfäche mit sogenannten Nanobuckeln beschichtet sein. Eine solche nutzt die Adhäsionswirkung, welche die Füsse von Geckos entfalten, welche echsenartigen Tiere ja bekanntlich auch an polierten Glaswänden oder Decken gehen können, indem ihre Füsse direkt in die molkulare Struktur der Wand eingreifen. Die Füsse dieser Tiere weisen Millionen feinster Häärchen an den Fusssohlen auf, von denen sich jedes bis zur Spitze in bis zu tausend winzige Knospen auffächert. Der bürstenartige Aufbau der Häärchen bringt die Gecko-Füsse so eng mit dem Untergrund in Kontakt, dass Anziehungskräfte wirken, die gewöhnlich nur zwischen einzelnen Atomen und Molekülen herrschen, die sogenannten Van-der-Waals-Kräfte, das heisst Kräfte aufgrund von elektrostatischen Wechselwirkungen zwischen Atomen oder Molekülen, wenn die Abstände nurmehr wenige Atomdurchmesser betragen. An der University of Califomia in Berkeley gelang es, Gummistücke mit einer ähnlichen Oberfläche herzustellen, die mit sogenannten Nanobuckeln versehen ist. (Proceedings of the National Academy of Sciences, Online-Publikation, vom 27. August 2002; www.pnas.org/cgi/content/abstract/192252799v1).

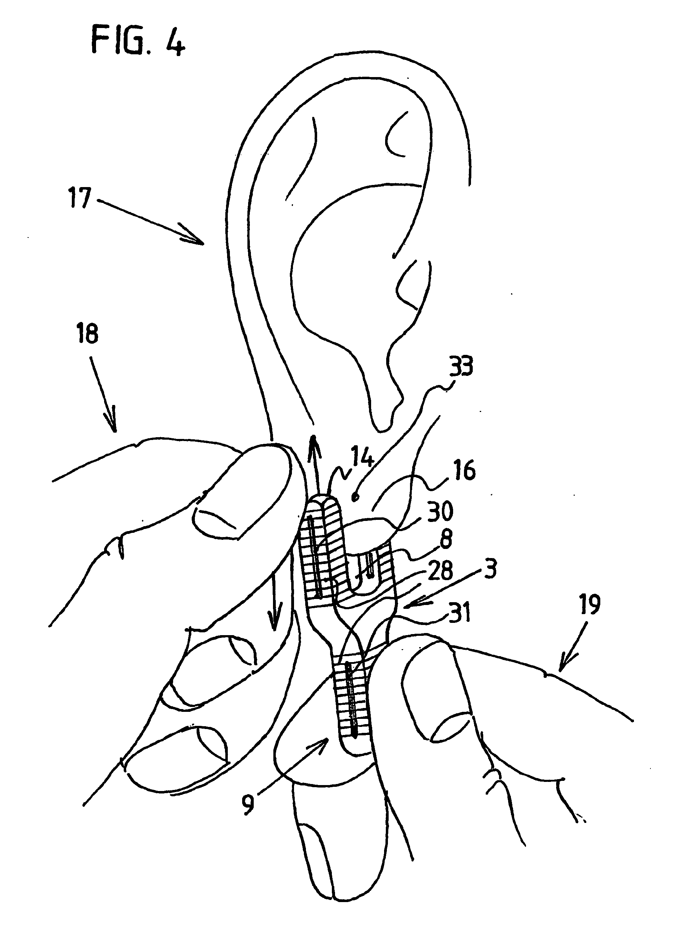

In Figur 4 ist dargestellt, wie die Dicke eines Ohrläppchens 16 mit Hilfe einer Einzellehre 3 vermessen wird. Hierzu ergreift - wie hier an einem rechten Ohr 17 gezeigt - die linke Hand 18 mit Zeigfinger und Daumen das Ohrläppchen 16 und zieht es wie mit dem Pfeil angedeutet nach unten, sodass es sich dehnt und dadurch dünner wird. Dann schiebt die rechte Hand 19 eine Einzellehre 3 mit ihrem Maul 8 bzw. ihrer U-förmigen Ausnehmung bzw. deren Enden 14 voraus über das in dieser Weise gedehnte Ohrläppchen 16. Es wird nun einfach eine solche Einzellehre 3 gewählt, deren Maul 8 gerade noch über das gedehnte Ohrläppchen 16 schiebbar ist. Lässt die linke Hand 18 das Ohrläppchen 16 los, so kann sich dieses wieder zurückziehen und seine Dicke schwillt ein wenig an. Danach wird die Einzellehre 3 passgenau und sicher am entspannten Ohrläppchen 16 gehalten. Mit einem Ziehen an der Einzellehre 3 in Richtung nach unten kann die Festigkeit des Sitzes geprüft werden. Die Skalierung 28 erlaubt nun festzustellen, wie weit das Maul 8 der Einzellehre 3 über das Ohrläppchen 16 gestülpt werden kann, denn nicht jedes Ohrläppchen ist gleichlang. Ausserdem kann eine Nadel durch den Schlitz 30 geschoben werden, sodass sie durch das im Ohrläppchen gegebenenfalls bereits vorhandene Loch 33 gestossen werden kann. Die Lage der Nadel kann hernach an der Skalierung abgelesen werden, sodass am Ohrschmuck später genau definiert werden kann, wo eine Sicherungsnadel positioniert werden muss. Sind diese Ausmess-Schritte vollzogen, zieht man mit einer Hand das Ohrläppchen wieder nach unten, sodass es sich dehnt und mit der anderen Hand kann dann die Einzellehre 3 einfach wieder vom Ohrläppchen 16 abgezogen werden.

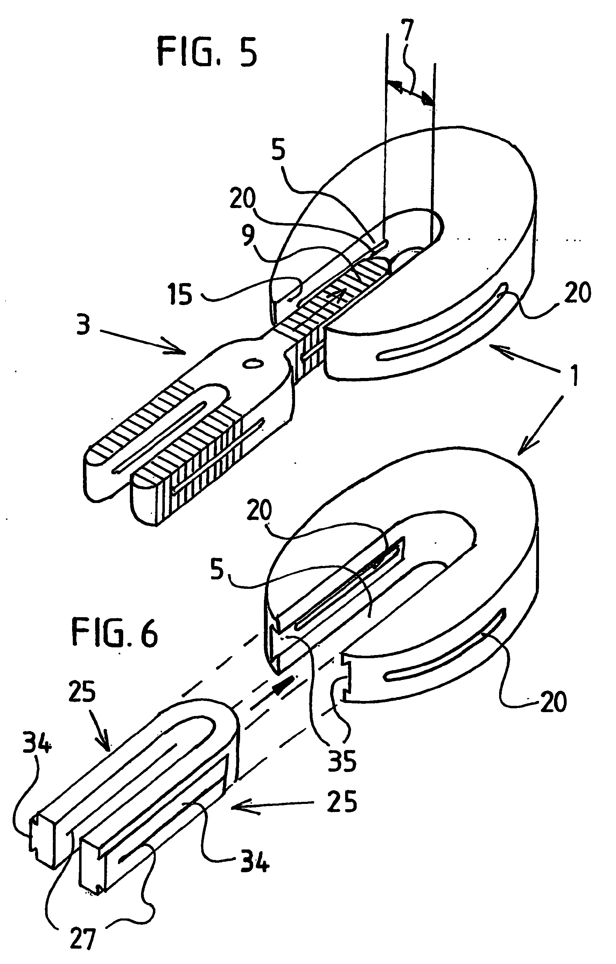

Die Figur 5 zeigt wie nun diese Einzellehre 3 zur Bestimmung des zur ausgemessenen Dicke des Ohrläppchens passenden Ohrschmuck-Stücks dient. Man benutzt hierzu ihre Zunge 9 zum Herausfinden der richtigen Grösse des Ohrschmuckstückes, bzw. der richtigen lichten Weite 7 der Ausnehmung 5 an einem solchen Ohrschmuckstück 1 nach diesem System oder zur Bestimmung einer Einlage 25 mit der richtigen Weite, welche je nach Ausführung in die Ausnehmung 5 am Ohrschmuck einsteckbar ist. Mit Vorteil ist die U-förmige Ausnehmung 5 am Ohrschmuckstück 1 mit einer gummi- oder gummielastischen Folie 15 ausgekleidet oder aber ihre Innenflächen sind durch eine Ätzung gerauht, sodass sie von ähnlicher Rauheit wie die Reibfläche einer Zündholzschachtel sind. Die so beschichtete oder behandelte Ausnehmung 5 verschiedener Ohrschmuckstücke 1 wird nun über die Zunge 9 der als passend befundenen Einzellehre 3 geschoben, wobei die Ausnehmung 5 gerade noch über die Zunge 9 geschoben werden können muss. Wenn das zutrifft, ist das passende Ohrschmuckstück gefunden. Es wird hernach an das Ohrläppchen angelegt, indem wiederum mit der einen Hand das Ohrläppchen nach unten gezogen wird, sodass es gedehnt wird und das Schmuckstück 1 mit seiner Ausnehmung 5 darüber geschoben wird. Das Schmuckstück 1 wird als im gedehnten Zustand des Ohrläppchens über dasselbe gestülpt, und hernach wird das Ohrläppchen losgelassen, sodass die Zugspannung entfällt und es in seine entspannte Lage zurückgeht, unter leichter Quillung. Damit ist ein sicherer und höchst bequemer Passsitz des in dieser Weise angelegten Ohrschmuckes gewährleistet. Wenn die Oberflächen der Innenseiten der Ausnehmung anstelle einer Kunststoff-Einalge zusätzlich mit Widerhaken wie in Figur 1 eingezeichnet versehen sind, so hält der Ohrschmuck noch sicherer, weil dann das aufquillende Ohrläppchen sich an diese Widerhaken anschmiegt.

In Figur 6 ist ein Ohrschmuckstück 1 mit einer zugehörigen Einlage 25 dar gestellt, die ihrerseits diejenige Weite aufweist, welche der gemessenen Ohrläppchendicke entspricht. Die Einlage 25 weist hier Federn 34 mit einem schwalbenschwanzförmigen Profil auf, welche in ebensolche Nuten 35 in der Ausnehmung 5 am Schmuckstück 1 einpassen, sodass die eingeschobene Einlage 25 in der Ausnehmung 5 kräftig gehalten ist. Anstelle von Nuten 35 und Federn 34 können auch Löcher und Noppen dienen, die ineinander eingreifen, oder seitliche Widerhaken an der Einlage 25 und der Ausnehmung 5, die beim Einschieben der Einlage 25 ineinander einrasten. Der Ohrschmuck 1 ist hier von einem horizontalen Schlitz 20 von ca. 1 mm Breite durchsetzt, welcher zum Einstecken einer Sicherungsnadel dient, denn Sicherungsnadeln weisen in der Regel eine Dicke von 0.8mm auf. Auch die zugehörige Einlage 25 ist von einem solchen horizontalen Schlitz 26 durchsetzt, der allerdings wegen der gummielastischen Beschaffenheit der Einlage geschlossen ist und nur beim Einstecken der Sicherungsnadel durch dieselbe geöffnet wird, wie das noch erklärt wird. Die Innenseiten der Ausnehmung an der Einlage 25 können mit Widerhaken versehen sein, ähnlich wie Figur 1 schon dargestellt, um ihren Halt an einem Ohrläppchen zu steigern.

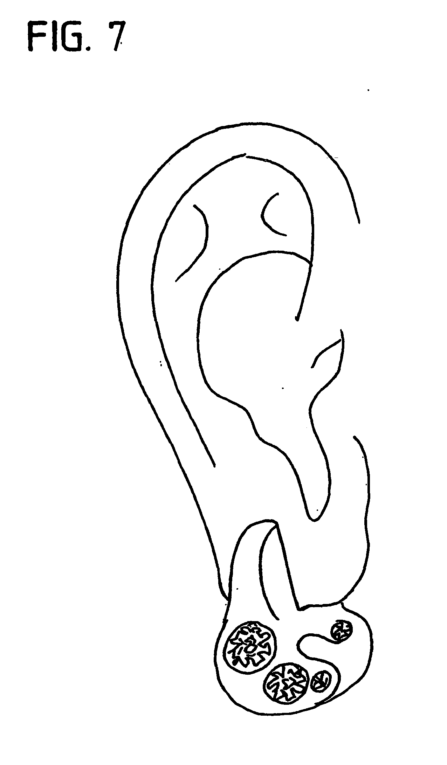

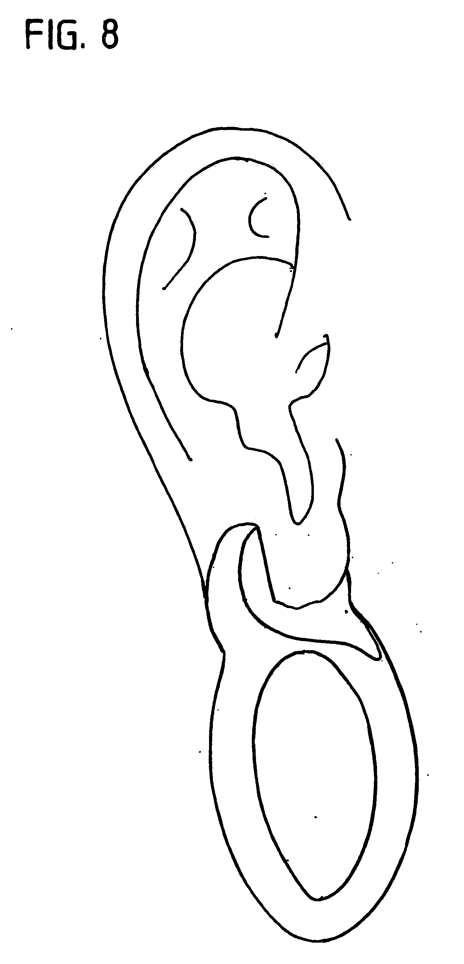

Die Figur 7 zeigt ein in dieser Weise auf ein Ohrläppchen aufgeschobenes Ohrschmuckstück. Wesentlich ist also die Ausnehmung am Schmuckstück sowie die Weite dieser Ausnehmung oder aber die Weite einer in dieselbe eingeschobenen Einlage. Diese Weite wird ganz individuell an die Dicke eines Ohrläppchen angepasst und wird grundsätzlich am durch Zug gedehnten Ohrläppchen gemessen und bestimmt. Der in der Figur untere Teil des Ohrschmucks kann nach Belieben geformt und gestaltet werden. Im hier gezeigten Beispiel ist der Ohr schmuck insgesamt glockenförmig gestaltet und er ist mit vier Steinen bestückt. Ebensogut kann ein Ohrschmuck auch als Kreole ausgebildet sein, wie das in Figur 8 gezeigt ist, oder er kann als Halterung zum Anhängen des eigentlichen Ohrschmuckes ausgebildet sein.

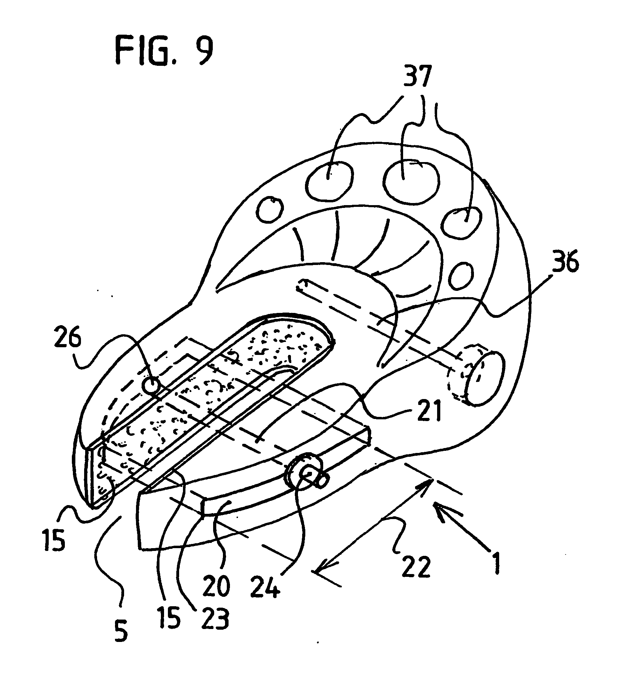

Für echten Ohrschmuck dient die Ausführung nach der Figur 9. Dieser Ohrschmuck 1 kann zum Beispiel aus Titan gefertigt sein und ist dann besonders leicht. Andrerseits kann er auch aus Gold oder Platin gefertigt sein oder aus Silber hergestellt und hernach vergoldet sein und mit Brillanten 37 bestückt sein. In diesem Falle ist er besonders wertvoll. Bei solchem Ohrschmuck ist es ratsam, ihn nicht allein durch eine Adhäsionswirkung am Ohrläppchen zu tragen, sondern ihn zusätzlich gegen ein Abstreifen oder Abrutschen zu sichern. Für diesen Zwecke ist der Ohrschmuck von der einer Seite her mit einem taschenförmigen Schlitz 20 versehen, wie das schon zum Ohrschmuck in Figur 6 erwähnt wurde, und welcher sich über die Ausnehmung 5 hinaus erstreckt und dort eine Tasche bildet, wie das strichliniert angedeutet ist. Die Innenwände der Ausnehmung 5 sind mit einer gummielastischen, schaumstoffartigen Folie 15 beschichtet. Dieser Ohrschmuck kann am Ohrläppchen gesichert werden, falls die Ohrläppchen der Trägerin gelocht sind, was ja bei sehr vielen Damen zutrifft oder im Bedarfsfall jederzeit nachgeholt werden kann. Doch im Vergleich zu herkömmlichem Schmuck, welcher an gelochten Ohrläppchen getragen wird, funktioniert die Halterung dieses Ohrschmuckes ganz anders. Beim herkömmlichen Schmuck nämlich hängt das Gewicht des Schmuckes wesentlich an der Nadel, welche durch das Loch im Ohrläppchen gesteckt wird. Trägt jemand dauerhaft relativ schweren Ohrschmuck und das über viele Jahre, so werden die Löcher im Ohrläppchen ausgetragen und immer grösser, und die Ohrläppchen werden oft unförmig. Bei der hier vorgeschlagenen Lösung jedoch dient die Nadel 21 einzig der Sicherung des Schmuckes, nicht aber für dessen dauerhaftes Halten am Ohrläppchen. Der Ohrschmuck wird nämlich in der weiter oben beschriebenen Art an das durch Zug gedehnte Ohrläppchen angepasst und zum Anlegen bloss mit seiner U-förmigen Ausnehmung von unten. über das mit der einen Hand gedehnte Ohrläppchen gestülpt, und zwar mit der Seite mit dem Schlitz 20 gegen den Kopf hin gewandt. Nach Loslassen des Ohrläppchens, das heisst nach Aufgabe der Zugspannung formt sich das Ohrläppchen zurück und quillt etwas, wodurch der Ohrschmuck hinreichend gehalten wird. Im Prinzip könnte der Ohrschmuck bereits so getragen werden. Als Sicherung wird nun jedoch zusätzlich eine Nadel 21 mit einem Griffkopf 24, der selbst als Schmuck ausgebildet sein kann, von der offenen Seite des Schlitzes 20 her durch die Folie 15 und durch die Ausnehmung 5 hindurch und somit durch das Loch im Ohrläppchen und hernach erneut durch die jenseits der Ausnehmung 5 liegende Folie 15 und dann in den dort verbleibenden Schlitz 20 gesteckt. Die Nadel 21 kann hierzu irgendwo über den ganzen Querbereich 22 des Schlitzes 20 angeordnet sein, je nach Lage des Loches im Ohrläppchen. Die genaue Lage der Nadel 21 wird vorgängig ebenfalls mit Hilfe der Einzellehren bestimmt. Hierzu weisen diese Einzellehren ja ebenfalls Schlitze 30,31 auf, sowie eine seitliche Skalierung 28 mit feinen Strichen 29. Bei aufgeschobener Einzellehre 3 wird die Sicherungsnadel 21 durch das vorhandene Loch im Ohrläppchen gesteckt und an der Skalierung 28 kann ihre Lage abgelesen werden. Nun wird die Einzellehre 3 in die Ausnehmung 5 am Schmuckstück 1 gesteckt und an der gemessenen Stelle kann dann eine Bohrung durch das Schmuckstück gelegt wer den oder, wenn dieses über einen durchgehenden Schlitz 20 verfügt, kann die richtige Einsteckstelle markiert werden und hernach die Nadel 21 an dieser Stelle durch den Schlitz 20 und durch die Folie 15 gesteckt werden. Die zum Schmucksystem gehörige Nadel 21 weist vorteilhaft eine kugelförmige Spitze 26 auf. Diese dehnt beim Durchstossen den Schlitz in der Folie 15 auf und verhindert hernach das Herausrutschen der Nadel 21, indem die kugelförmige Spitze 26 als Widerhaken wirkt. Das Gleiche trifft zu, wenn die Ausnehmung 5 am Schmuckstück mit einer Einlage 25 von der ausgemessenen Weite bestückt ist, die ihrerseits Schlitze 27 zum Durchstecken der Sicherungsnadel 21 aufweist, wie in Figur 6 gezeigt. Der Vorteil dieses Systems mit diesem Schlitz 20 liegt auch darin, dass die Nadel 21 gewissermassen stufenlos innerhalb des Schlitzes 20 platziert werden kann, das heisst die Lage der Nadel 21 am Schmuckstück kann genau an das Loch im Ohrläppchen einer Trägerin angepasst werden. In jeder Lage sticht die Nadel 21 durch die Folie 15 oder die Einlage 25 und das Schmuckstück ist somit gesichert. Der in dieser Weise mit der Nadel 21 gesicherte Schmuck 1 hängt beim Tragen jedoch nur an der Ausnehmung 5. Auf die Nadel 21 wirken keine Kräfte. Sie kommt nur dann zum Tragen, wenn der Schmuck 1 mit grösserer Kraftwirkung als bloss seinem Eigengewicht vom Ohrläppchen gezogen wird. Dann bleibt das Schmuckstück 1 an der Nadel 21 hängen, nachdem sich diese zunächst an das vordere Ende 23 des Schlitzes 20 verschoben hat. Diese Nadel 21 erlaubt sogar eine Höhenverstellbarkeit des Ohrschmuckes an einem Ohrläppchen. Die Nadel 21 ist nämlich an jeder beliebigen Stelle innerhalb des Schlitzes 20 durch die schaumstoffartige Folie 15 oder die Schlitze 27 an der Einlage 25 steckbar. Entsprechend kann ein Ohrschmuck nur zum Teil mit seiner Ausnehmung 5 über ein Ohrläppchen geschoben werden und die Nadel wird dann dort durch die schaumstoffartige Folie 15 oder die Einlage 25 gesteckt, wo sich das Loch im Ohrläppchen befindet. Wird der Ohrschmuck so angelegt, so wird er nur zum Teil von der Ausnehmung 5 infolge Adhäsionskraft gehalten, und ein Teil der Haltekraft wird von der Nadel 21 übernommen, welche ja zweimal durch die an das Ohrläppchen angrenzenden Folien 15 bzw. durch die Einlage 25 geführt ist. Diese Folie 15 oder Einlage 25 kann übrigens auch als Schmuck ausgebildet sein, indem sie die Ausnehmung seitlich überragt und dort entsprechende Formen annimmt, sodass sie dekorativ wirkt. Das Schmuckstück selbst kann eine zusätzliche Bohrung 36 aufweisen, in welcher die Sicherungsnadel 21 bei Nichtgebrauch eingesteckt und versorgt werden kann. Sie kann dann bei Bedarf, etwa wenn bei Ausübung einer sportlichen Tätigkeit zusätzliche Kräfte auf den Ohrschmuck wirken, als Sicherung angelegt werden, während sie im Normalfall gar nicht nötig ist.

Dieser Ohrschmuck und sein System erlauben ein besonders bequemes und vor allem schmerzfreies Tragen von Ohrschmuck, insbesondere von Modeschmuck, welcher sonst und herkömmlich mittels Klemm-Mechanismen an den Ohrläppchen befestigt wird. Das System erlaubt aber auch die Ausführung von echtem Ohrschmuck unter zusätzlicher Sicherung mittels einer Nadel, die jedoch beim normalen Tragen nicht belastet ist und daher auch ein Loch im Ohrläppchen nicht beansprucht oder dehnt. Der Ohrschmuck kann auch aus einer Halterung bestehen, welche nach diesem System funktioniert, und an welcher dann der eigentliche Ohrschmuck hängt. Von entscheidender Bedeutung für die korrekte Funktion des Systems ist es, dass die Ohrläppchendicke im durch nach unten Ziehen gedehnten Zustand des Ohrläppchens bestimmt wird, und der Schmuck in diesem gedehnten Zustand mit der entsprechend ausgemessenen Weite der Ausnehmung über das Ohrläppchen gestreift wird. Dann erst wird das Ohrläppchen losgelassen, wodurch es sich unter Aufquillung zurückformt und dann den Ohr schmuck infolge von Adhäsionskraft sicher hält.

Einzellehren 3 der Lehre 2 können mit standardisierten Grössen eingesetzt werden, sodass ein international gültiger Standard für deren lichte Weiten und für deren Skalierung geschaffen wird. Dieser Standard würde es erlauben, dass bei bekannter Ohrläppchendicke in gedehnten Zustand, das heisst bei bekannter lichter Weite der Lehre und des Ohrschmuckes sowie bei bekannter Lage der Sicherungsnadel anhand des Skalenwertes ein Ohrschmuck ohne Anpropieren gekauft werden kann und dann auf Anhieb perfekt sitzt.

## Patentansprüche

1. Ohrschmuck-System mit zugehöriger Lehre (2) aus Einzellehren (3) zur optimalen Anpassung des Ohrschmucks (1) an das Ohrläppchen, ***dadurch gekennzeichnet,* dass** der Ohrschmuck eine U-förmige Ausnehmung (5) aufweist, welche dazu bestimmt ist, mit ihrer lichten Weite (7) oder mit der lichten Weite einer in sie einzulegenden Einlage (25) von unten über das zu bestückende, für die Zwecke des Anlegens gedehnte Ohrläppchen (16) geschoben zu werden und dabei genau die Dicke dieses gedehnten Ohrläppchens (16) aufzunehmen, wobei die passende lichte Weite (7) mittels einer zum System gehörige Lehre (2) aus einer Mehrzahl von Einzellehren (3) mit je Mäulern (8) von abgestuften lichten Weiten (12) und Zungen (9) der Dicke (13) der jeweiligen lichten Weiten (7) bestimmbar ist, sodass anhand einer Messung eines zu bestückenden Ohrläppchens (16) im gedehnten Zustand mittels dieser Einzellehren (3) die passende lichte Weite (7) der U-förmigen Ausnehmung (5) des zugehörigen Ohrschmucks (1) oder der einzulegenden Einlage (25) aus einer Ohrschmuck-Reihe oder Einlagen-Reihe mit U-förmigen Ausnehmungen (5) unterschiedlicher lichter Weiten bestimmbar ist.

2. Ohrschmuck-System nach Anspruch 1, ***dadurch gekennzeichnet,* dass** die zum System und dessen Lehre (2) gehörigen Einzellehren (3) eine einheitliche Skalierung (28) an ihren Mäulern (8) und an ihren Zungen (9) aufweisen, zur Messung der Überstülptiefe an einem Ohrläppchen (16) und zur Bestimmung der Lage eines allfällig vorhandenen Loches (33) im Ohrläppchen (16).

3. Ohrschmuck-System nach einem der vorangehenden Ansprüche, ***dadurch gekennzeichnet,* dass** die Innenwände (6) der U-förmigen Ausnehmungen (5) am Ohrschmuck (1) mit ein oder mehreren länglichen Widerhaken versehen sind, welche zum Verkrallen mit dem Ohrläppchen oder zur Aufnahme und Rückhaltung einer Einlage (25) bestimmt sind.

4. Ohrschmuck-System nach einem der Ansprüche 1 bis 2, ***dadurch gekennzeichnet,* dass** die Innenwände (6) der U-förmigen Ausnehmungen (5) am Ohrschmuck (1) mit einer gummielastischen Folie (15) beschichtet sind oder in die U-förmigen Ausnehmungen (5) gummielastische Einlagen (25) mit unterschiedlichen lichten Weiten mittels Nuten und Federn oder Löchern und Noppen einsteckbar sind.

5. Ohrschmuck-System nach einem der vorangehenden Ansprüche, ***dadurch gekennzeichnet,* dass** die U-förmige Ausnehmungen (5) am Ohrschmuck (1) von einem taschenförmigen Schlitz (20) durchsetzt sind, welcher von der einen Seite des Ohrschmucks (1) aus demselben ausgenommen ist und sich quer bis über die Ausnehmung (5) hinaus erstreckt, sowie dass zum Ohrschmuck (1) eine Sicherungsnadel (21) mit Griffkopf (24) gehört, welche in den Schlitz (20) einpasst und eine kugelförmige Spitze (26) aufweist.

6. Ohrschmuck-System nach einem der vorangehenden Ansprüche, ***dadurch gekennzeichnet,* dass** die gummielastische Folie (15) in der U-förmigen Ausnehmung (5) am Ohrschmuck (1) oder die eingesteckte Einlagen (25) mit geschlossenen Schlitzen (27) ausgerüstet ist, die sich längs des taschenförmigen Schlitzes (20) erstrecken und zum Durchstossen mit der Sicherungsnadel (21) bestimmt sind, wobei deren kugelförmige Spitze (26) als Widerhaken wirkt, sodass die Sicherungsnadel (21) vor einem Herausgleiten gesichert ist.

7. Ohrschmuck-System nach einem der vorangehenden Ansprüche, ***dadurch gekennzeichnet,* dass** der Ohrschmuck (1) eine Bohrung (36) zur Aufnahme der Sicherungsnadel (21) bei deren Nichtgebrauch aufweist.

8. Ohrschmuck-System nach einem der vorangehenden Ansprüche, ***dadurch gekennzeichnet,* dass** der Ohrschmuck (1) aus einem Edelmetall, aus Edelstahl oder Titan gefertigt ist und mit Edelsteinen besetzt ist.

9. Ohrschmuck-System nach einem der Ansprüche 1 bis 6, ***dadurch gekennzeichnet,* dass** der Ohrschmuck (1) aus Kunststoff, Holz oder aus Glas gefertigt ist.

10. Ohrschmuck-System nach einem der Ansprüche 1 bis 6, dass die Ausnehmung (5) mit einer Gummischicht mit einer Oberfläche mit Nanobuckeln beschichtet ist.

## Claims

1. Ear jewellery system with associated gauge (2) comprising of separate gauges (3) for optimal adaptation of the ear jewellery (1) on the ear lobe, **characterized in that** the ear jewellery has a U shaped recess (5), which, with its internal width (7) or with the internal width of an insert (25), to be put in the said recess, is intended for being pushed over the ear lobe (16) from below, the said ear lobe to be fitted, being stretched for the purpose of putting on, and thereby taking in precisely the thickness of this stretched ear lobe (16), wherein the suitable internal width (7) is determined by means of an appropriate gauge (2) for the system comprising of plurality of separate gauges (3) with respective mouths (8) of graded internal widths (12) and tongues (9) having thickness (13) of respective internal widths (7), so that with the help of a measurement of an ear lobe (16) to be fitted, in stretched condition, by means of these separate gauges (3), the suitable internal width (7) can be ascertained of the U shaped recess (5) of the relevant ear jewellery (1 ) or of the insert (25) to be put in, from a series of ear jewellery or series of inserts with U shaped recess (5), having different internal widths.

2. Ear jewellery system as per Claim 1, **characterized in that** the separate gauges (3), belonging to the system and its gauge (2) have an integral scale (28) on their mouths (8) and on their tongues (9), for measuring the depth of putting on to an ear lobe (16) and determining the position of a possible hole (33) present in the ear lobe (16).

3. Ear jewellery system as per any of the previous Claims, **characterized in that** the inner walls (6) of the U shaped recesses (5) on the ear jewellery (1) are provided with one or several longish barbs, which are intended for digging in with the ear lobe or for taking in and supporting an insert (25).

4. Ear jewellery system as per any of the Claims 1 to 2, **characterized in that** the inner walls (6) of the U shaped recesses (5) on the ear jeweilery (1) are coated with a rubber like foil (15) or rubber like inserts (25) with different internal widths can be put in the U shaped recesses (5) by means of grooves and springs or holes and knops.

5. Ear jewellery system as per any of the previous Claims, **characterized in that** the U shaped recesses (5) on the ear jewellery (1) are made with a pocket shaped slit (20), which is taken out from itself from one of the sides and extends across and over the recess (5), as well as that a safety needle (21) with grip head (24) belongs to the ear jewellery, the said needle fitting in the said slit (20) and has a ball shaped tip (26).

6. Ear jewellery system as per any of the previous Claims, **characterized in that** the rubber like foil (15) in the U shaped recess (5) on ear jewellery (1) or the insert put in with closed slits (27) is equipped, extending along the pocket shaped slit (20) and is intended to be penetrated by the safety needle (21), wherein the ball shaped tip (26) of the said needle works as barb, so that the safety needle is secure against slipping off.

7. Ear jewellery system as per any of the previous Claims, **characterized in that** the ear jewellery (1) has a hole (36) for taking in the safety needle (21) when it is not in use.

8. Ear jewellery system as per any of the previous Claims, **characterized in that** the ear jewellery (1) is made from a precious metal, stainless steel or titanium and is fitted with precious stones.

9. Ear jewellery system as per any of the Claims 1 to 6, **characterized in that** the ear jewellery (1) is made from plastic, wood or glass.

10. Ear jewellery system as per any of the Claims 1 to 6, **characterized in that** the recess (5) is coated with a rubber layer, having a surface with nano humps.

## Revendications

1. Système de bijou d'oreille avec calibre associé (2) constitué de calibres individuels (3) pour l'adaptation optimale du bijou d'oreille (1) au lobe d'oreille, ***caractérisé en* ce que** le bijou d'oreille présente un évidement en forme de U (5) qui, avec sa largeur intérieure (7) ou avec la largeur intérieure d'un insert (25) devant être placé dedans, est destiné à être poussé par le bas sur le lobe d'oreille (16) à garnir et étiré dans le but de la mise en place, et d'épouser alors exactement l'épaisseur de ce lobe d'oreille étiré (16), sachant que la largeur intérieure adéquate (7) peut être déterminée au moyen d'un calibre (2) appartenant au système parmi une pluralité de calibres individuels (3), munis chacun de découpures (8) comportant des largeurs intérieures échelonnées (12) et de languettes (9) dont l'épaisseur (13) correspond aux largeurs intérieures respectives (7), de telle sorte qu'il est possible de déterminer la largeur intérieure adéquate (7) de l'évidement en forme de U (5) du bijou d'oreille associé (1) ou de l'insert (25) devant être placé dedans à partir d'une série de bijoux d'oreille ou d'une série d'inserts présentant des évidements en forme de U (5) de différentes largeurs intérieures, à l'aide d'une mesure d'un lobe d'oreille à garnir (16) dans l'état étiré au moyen de ces calibres individuels (3).

2. Système de bijou d'oreille selon la revendication 1, ***caractérisé en* ce que** les calibres individuels (3) appartenant au système et à son calibre (2) présentent un graduation uniforme (28) sur leurs découpures (8) et sur leurs languettes (9) pour mesurer la profondeur de recouvrement sur un lobe d'oreille (16) et pour déterminer la position d'un perçage éventuellement présent (33) dans le lobe d'oreille (16).

3. Système de bijou d'oreille selon l'une des revendications précédentes, ***caractérisé en* ce que** les parois intérieures (6) des évidements en forme de U (5) sur le bijou d'oreille (1) sont munies d'une ou de plusieurs barbes allongées qui sont destinées à s'accrocher au lobe d'oreille ou à réceptionner et à retenir un insert (25).

4. Système de bijou d'oreille selon l'une des revendications 1 à 2, ***caractérisé en* ce que** les parois intérieures (6) des évidements en forme de U (5) sur le bijou d'oreille (1) sont enduites d'un film élasto-caoutchouté (15) ou **en ce que** des inserts élasto-caoutchoutés (25) peuvent être introduits dans les évidements en forme de U (5) avec des largeurs intérieures différentes au moyen de rainures et de ressorts ou de trous et de noppes.

5. Système de bijou d'oreille selon l'une des revendications précédentes, ***caractérisé en* ce que** les évidements en forme de U (5) sur le bijou d'oreille (1) sont entrecoupés d'une fente en forme de poche (20), laquelle est évidée depuis un des côtés du bijou d'oreille (1) depuis celui-ci et s'étend transversalement jusqu'au-delà de l'évidement (5), ainsi qu'en ce qu'une aiguille de sûreté (21) avec tête de saisie (24) appartient au bijou d'oreille (1), laquelle s'encastre dans la fente (20) et présente une pointe (26) en forme de bille.

6. Système de bijou d'oreille selon l'une des revendications précédentes, ***caractérisé en* ce que** le film élasto-caoutchouté (15) dans l'évidement en forme de U (5) sur le bijou d'oreille (1) ou les inserts introduits (25) est (sont) équipé(s) de fentes fermées (27) qui s'étendent le long de la fente en forme de poche (20) et qui sont destinées à la percée avec l'aiguille de sûreté (21), leur pointe (26) en forme de bille agissant comme barbe, de telle sorte que l'aiguille de sûreté (21) est assurée contre un glissement vers le dehors.

7. Système de bijou d'oreille selon l'une des revendications précédentes, ***caractérisé en* ce que** le bijou d'oreille (1) présente un perçage (36) pour réceptionner l'aiguille de sûreté (21) lorsqu'elle n'est pas utilisée.

8. Système de bijou d'oreille selon l'une des revendications précédentes, ***caractérisé en* ce que** le bijou d'oreille (1) est fabriqué à partir d'un métal précieux, d'acier surfin ou de titane et qu'il est garni de pierres précieuses.

9. Système de bijou d'oreille selon l'une des revendications 1 à 6, ***caractérisé en* ce que** le bijou d'oreille (1) est fabriqué à partir de matière plastique, de bois ou de verre.

10. Système de bijou d'oreille selon l'une des revendications 1 à 6, ***caractérisé en* ce que** l'évidement (5) est enduit d'une couche de caoutchouc avec une surface dotée de nano-bosses.
